# EUROPÄISCHE PATENTSCHRIFT

(11) **EP 0 356 896 B1**
(45) Veröffentlichungstag und Bekanntmachung des Hinweises auf die Patenterteilung: **05.01.1994**
(21) Anmeldenummer: 89115527.7
(22) Anmeldetag: 23.08.1989
(51) Int. Cl.: A61L 2/10

(54) **Vorrichtung zum Entkeimen von Toiletteninstrumenten oder ärztlichen Instrumenten**
Sterilizing device for toilet instruments or medical instruments
Dispositif pour la stérilisation d'instruments de toilette ou d'instruments médicaux

(30) Priorität: 24.08.1988 CH 3147/88
(43) Veröffentlichungstag der Anmeldung: 07.03.1990
(73) Patentinhaber: AMESEDER, Anton, CH-9400 Rorschach (CH)
(72) Erfinder: AMESEDER, Anton, CH-9400 Rorschach (CH)
(74) Vertreter: Riebling, Peter, Dr.-Ing.

(56) Entgegenhaltungen:
- WO-A-83/03202
- WO-A-88/06042
- US-A- 3 748 094

## Beschreibung

Die vorliegende Erfindung betrifft eine Vorrichtung zum Entkeimen von Toiletteninstrumenten oder ärztlichen Instrumenten oder dergleichen, insbesondere Zahnbürsten durch ultraviolette Strahlung, wobei die Instrumente bzw. Zahnbürsten in einem Gehäuse in Verbindung mit einer Grundplatte angeordnet sind und von einer Lichtquelle, die UV-Strahlung erzeugt, angestrahlt werden.

Die Desinfektion, d.h. das Unschädlichmachen von Krankheitserregern, insbesondere von Bakterien, erfolgte bisher meist durch chemische Mittel. Ebenfalls seit langem bekannt ist das Sterilisieren durch Hitzeeinwirkung, wobei Autoklaven verwendet werden, die mit Temperaturen von 120° bis 145° C unter Dampfdruck arbeiten.

Aus der DE-OS 32 09 701 ist es auch bereits schon bekannt, insbesondere auch ärztliche Instrumente mit Hilfe von UV-Strahlung keimfrei zu machen. Die ärztlichen Instrumente sind hierbei auf einer Grundplatte in Schlitze eingesteckt und durch Einstellen der Grundplatte in ein Gehäuse werden die Instrumente der UV-Strahlung zwecks Entkeimung ausgesetzt. Die gesamte Vorrichtung ist jedoch in der Handhabung relativ schwierig und weiterhin läßt sich keine genaue gleichmäßige Verteilung der UV-Strahlung über den gesamten Entkeimungsbereich erzielen. Die bekannte Vorrichtung ist außerdem nicht zum Entkeimen von Zahnbürsten geeignet.

Eine derartige Vorrichtung zum Entkeimen, insbesondere von Zahnbürsten ist aus der WO 8 806 042 der Anmelderin bereits offenbart. Die darin beschriebene Vorrichtung zum Entkeimen besteht aus einem kastenförmigen Gehäuse, auf dessen Grundplatte sich eine UV-Strahlungsquelle befindet. Die keimfrei zu machenden Instrumente werden dabei derart in eine einschiebbare Halterung gesteckt, daß diese der ultravioletten Strahlung der Lichtquelle ausgesetzt sind. Auch diese Vorrichtung besitzt eine schwierige Handhabbarkeit und ist außerdem schwierig zu reinigen und zu bedienen. Dabei ist insbesondere die Halterung aufwendig ausgebildet und daher teuer in der Herstellung.

Aufgabe der vorliegenden Erfindung ist es deshalb, bei gleichmäßiger Verteilung der UV-Strahlung eine einfach handhabbare und preisgünstig herstellbare Vorrichtung zum Entkeimen von Toiletteninstrumenten oder ärztlichen Instrumenten, insbesondere von Zahnbürsten zu schaffen.

Die Lösung der Aufgabe gelingt mit den Merkmalen des kennzeichnenden Teils des Patentanspruchs 1.

Das Wesen der Erfindung liegt darin, daß in stets wiederholbarer Weise an die Grundplatte bzw. die Strahlungsquelle die zu entkeimenden Instrumente bzw. Zahnbürsten angesetzt werden, wobei der Entkeimraum selbst von einem schwenkbaren Gehäuse umschlossen wird. Mit dem Ansetzen der Instrumente bzw. der Zahnbürsten an die Grundplatte bzw. UV-Lichtquelle über einen Aufnahmebehälter mit einem Aufnahmekamm wird eine stets reproduzierbare gleichmäßige UV-Ausstrahlung des angesetzten Aufnahmebehälters erreicht. Durch das Umschließen des Aufnahmebehälters mit einem Gehäuse, wobei die Instrumente bzw. Zahnbürsten in Öffnungen des Gehäuses eingesteckt sind, wird bei leichter Handhabbarkeit ein Entkeimungsraum geschaffen, der in unmittelbarer Nähe der UV-Lichtquelle einen besonders hohen Wirkungsgrad betreffend das Entkeimen der ärztlichen Instrumente bzw. der Zahnbürsten aufweist.

Die erfindungsgemäße Vorrichtung eignet sich deshalb besonders für Zahnbürsten, die sonst offen dem Staub und dem feuchtwarmen Klima ausgesetzt im Badezimmer in einem Trinkglas aufbewahrt werden oft noch zusammen mit anderen Gegenständen, die ebenfalls Keime enthalten, wie z. B. Naßrasierer und dergleichen. Bei dieser Aufbewahrung vermehren sich die Bakterien an beispielsweise so aufgestellten Zahnbürsten in wenigen Stunden millionenfach. Durch den Gebrauch der Zahnbürsten gelangen die Krankheitserreger in den Mund, wo sie wesentlich zu entzündlichen Erkrankungen im Zahn- und Rachenbereich beitragen.

Die Erfindung ist im weiteren auch vor allem im medizinischen Bereich anwendbar. Hier kann die Erfindung auf das Desinfizieren von Coiffeur-Instrumenten, wie Rasiermesser, Rasierklingen, Haarscheren oder Scherköpfen von Haarschneidemaschinen, angewendet werden.

In vorteilhafter Weise weist die Grundplatte der Vorrichtung zum Entkeimen eine Wand mit einer Öffnung für die UV-Lichtquelle auf, wobei der Aufnahmebehälter mit dem Aufnahmekamm nach Art eines Einschubes an die Lichtquelle herangeführt werden.

Hierdurch ergibt sich in Verbindung mit dem Einschieben des Aufnahmebehäters für die Instrumente bzw. Zahnbürsten eine stes gleichmäßige Ausleuchtung des Entkeimbereiches mit UV-Strahlung.

Vorteilhaft ist es hierbei vorgesehen, daß die Schenkel der Grundplatte Führungen aufweisen, in welche der Aufnahmebehälter über weitere Führungen einschiebbar ist. Durch das Einschieben des Aufnahmebehälters über Führungen an die Grundplatte kann der Aufnahmebehälter auf einfache Weise plaziert werden und kann im weiteren, etwa zu Reinigungszwecken, leicht von der Grundplatte wieder entfernt werden.

Der Boden des Aufnahmebehälters ist vorteilhaft wannenförmig mit einer Ablauföffnung ausgebildet, wodurch etwaige Reinigungsflüssigkeit an den Instrumenten oder Zahnbürsten unschädlich abtropfen kann.

Die wannenförmige Ausbildung des Aufnahmebehälters bringt auch den Vorteil, daß in Verbindung mit dem umschließenden Gehäuse im Bodenbereich der Vorrichtung ein vorteilhafert Entkeimungsraum geschaffen wird, wo sich zusätzlich für die Entkeimung vorteilhaft ein gewißer Ozonspiegel ansammelt. Dieser Ozonspiegel, der sich je nach Entkeimungsdauer vergrößert, wird hierbei in der Wanne des Aufnahmebehälters im Bereich des Aufnahmekamms für die ärztlichen Instrumente bzw. Zahnbürsten gehalten, wobei ein derartiger Ozonspiegel im besonderen durch das umschließende und einrastende Gehäuse am abfließen gehindert wird.

Durch die Ausbildung eines Aufnahmebehälters mit dem wannenförmigen Boden, der zusätzlich im Bereich der Grundplatte von einem schwenkbaren Gehäuse umschlossen ist, wird nach dem Wesen der Erfindung eine besonders vorteilhafte UV-Entkeimung erzielt, weil in mehrfacher Reflektion der UV-Strahlung sowohl im Bereich des Aufnahmebehälters als auch am Gehäuse eine schattenfreie UV-Bestrahlung der zu entkeimenden ärztlichen Instrumente bzw. der Zahnbürsten erreicht wird.

In vorteilhafter Ausbildung ist es vorgesehen, daß der Aufnahmebehälter eine hochgezogene Wand aufweist, wo in Verbindung mit dem eingeschobenen Aufnahmekamm ein Aufnahmespalt für Instrumente bzw. die Zahnbürsten gebildet wird.

In weiterer vorteilhafter Ausgestaltung sind an der Grundplatte in seitlicher Anordnung mit Ausleuchtung des Innenraumes des Gehäuses im Bereich des Aufnahmebehälters und der Öffnungen im Gehäuse Infrarotsensoren, bestehend aus Sender- und Empfängerdiode angeordnet.

Hierdurch schaltet sich die UV-Strahlungsquelle bei einem Bewegungsvorgang innerhalb des Gehäuses bzw. des Aufnahmebehälters automatisch ein, wodurch die Entkeimung eingeleitet wird.

Durch eine wissenschaltliche Untersuchung der erfindungsgemäßen Vorrichtung durch das Institut für Hygiene und Medizinische Mikrobiologie der Universität Bern wurde deren Wirksamkeit festgestellt. Aus einer Übernachtkultur von von Escherichia coli wurde eine Keimsuspension hergestellt, die rund 5 x 10⁶ Keime pro ml enthielt. In diese Keimsuspension wurde dann eine Reihe von Zahnbürsten eingetaucht und anschließend ausgeschlagen, um überschiessende Flüssigkeit mit Bakterien zu entfernen. Die so vorbehandelten Bürsten wurden dann entweder direkt in ein frisches und steriles Nährmedium gebracht, oder 10 Minuten, 20 Minuten oder 40 Minuten in die Vorrichtung bei brennender UV-Lichtquelle gestellt, wobei jeweils die Keimzahl in diesen Medien bestimmt wurde. Daraus ergab sich, daß nach 40 Minuten 98 % der Keime abgetötet waren.

Auf Grundlage dieser Versuche ist deshalb die Steuerungseinrichtung vorteilhaft mit einem Zähler ausgestattet, welcher die eingeschaltete UV-Lichtquelle nach einer vorgegebenen Zeit, beispielsweise nach einer Stunde, selbsttätig abschaltet.

In einer weiteren Ausbildung der Steuerungseinrichtung kann dieselbe auch mit einem Thermostat ausgestattet sein, der die eingeschaltete UV-Lichtquelle beim Überschreiten einer vorgegebenen Temperatur ausschaltet und bei deren Unterschreiten wieder einschaltet.

Weitere vorteilhafte Ausgestaltungen der erfindungsgemäßen Vorrichtung ergeben sich aus den weiteren Unteransprüchen.

Nachfolgend wird anhand der Zeichnungsfiguren ein Ausführungsbeispiel der Erfindung näher beschrieben.

Es zeigen:
- Figur 1:: eine perspektivische Ansicht der Vorrichtung;
- Figur 2:: das Blockschema der Elektronik und der Steuerungseinrichtung bei einer Ausführungsform;
- Figur 3:: eine perspektivische Ansicht der Grundplatte bei aufgeklapptem Gehäuse;
- Figur 4:: den Aufnahmebehälter in einer perspektivischen Ansicht;
- Figur 5:: den Aufnahmekamm der Vorrichtung;
- Figur 6:: den Aufnahmebehälter mit eingeschobenem Aufnahmekamm in einer Ansicht;
- Figur 7:: einen Querschnitt des Aufnahmebehälters mit eingeschobenem Aufnahmekamm nach Figur 6;
- Figur 8:: einen Querschnitt nach Figur 6 einer anderen Ausführungsform;
- Figur 9:: einen Querschnitt der Vorrichtung nach Figur 1, ohne Darstellung der eingeschobenen Zahnbürsten;
- Figur 10:: einen Längsschnitt der Grundplatte mit Darstellung der Elektronik in einem Aufnahmeschacht.

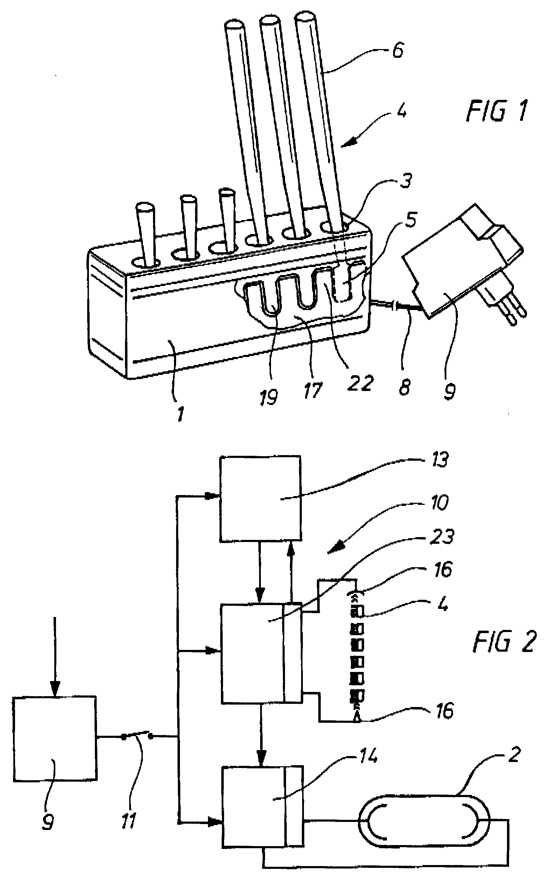

Die Vorrichtung zum Entkeimen besteht nach Figur 1 und den Figuren 3 bis 5 aus einem optisch ansprechenden Gehäuse, vorzugsweise aus Kunststoff, in welchem sich eine ultraviolette Strahlen erzeugende Lichtquelle 2 befindet. Im vorliegenden Fall handelt es sich um eine Röhre.

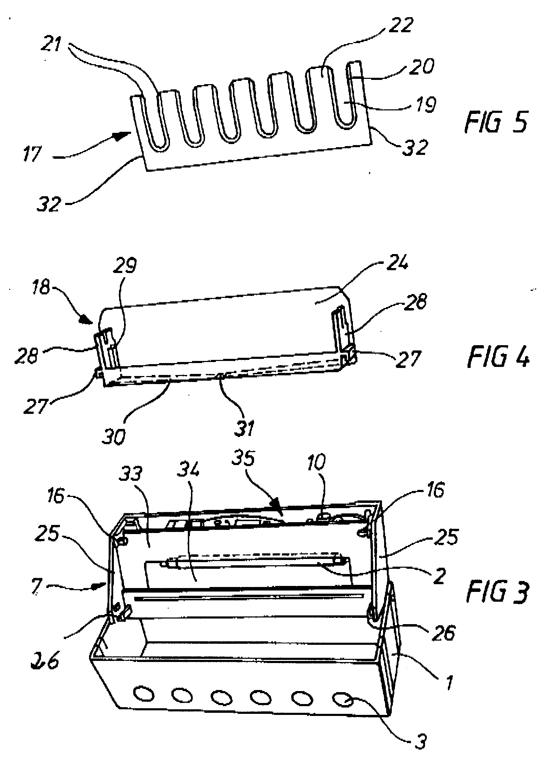

Das Gehäuse 1 weist nach Figur 1 mehrere Öffnungen 3 auf, in welche die Zahnbürsten 4 so einsteckbar sind, daß sich ihr Borstenkopf 5 im Gehäuse 1 befindet und ihr Griff 6 aus dem Gehäuse 1 herausragt. Die UV-Lichtquelle 2 ist nach Figur 3 auf der Grundplatte 7 des Gehäuses 1 befestigt und ist über ein Kabel 8 nach Figur 1 mit einem Stecknetzgerät 9 verbunden.

In Figur 1 ist das Gehäuse 1 an die Grundplatte 7 nach Figur 3 eingeschwenkt und eingerastet, wobei in den Öffnungen 3 Zahnbürsten 4, bestehend aus einem Borstenkopf 5 und einem Griff 6, eingeführt sind.

Aus Figur 1 ist weiterhin in teilweise Anordnung der Aufnahmekamm 17 ersichtlich, der Zinken 22 und dadurch gebildete Aufnahmeöffnungen 19 aufweist, wobei in eine Aufnahmeöffnung 19 ein Borstenkopf 5 einer Zahnbürste 4 eingeführt ist.

Die Figur 3 zeigt eine Darstellung ohne Aufnahmebehälter 18 und Aufnahmekamm 17 bei aufgeklapptem Gehäuse 1.

Die Grundplatte 7 weist hierbei seitliche Schenkel 25 auf, an denen Führungen 26 für den Aufnahmebehälter 18 nach Figur 4 angeordnet sind.

Weiterhin besteht die Grundplatte 7 aus einer Wand 33, in welcher eine Lichtöffnung 34 für die dahinter angeordnete Lichtquelle 2 angeordnet ist.

Seitlich an der Grundplatte 7 im oberen Bereich der spitz zulaufenden Schenkel 25 sind Sensoren 16 angeordnet, insbesondere Infrarotsensoren, bestehend aus Infrarotsender und Infrarotempfänger.

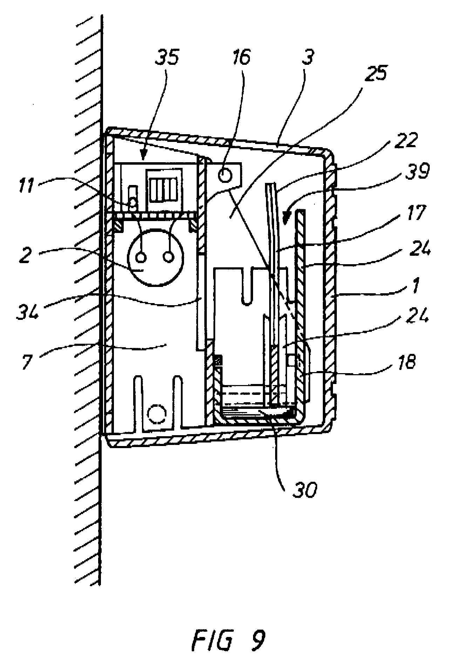

In Figur 3 ist in Verbindung mit Figur 9 auch noch der Aufnahmeschacht 35 der Grundplatte 7 ersichtlich, wo die gesamte Elektronik einschließlich der Steuerungseinrichtung 10 und der Lichtquelle 2 auf einer gemeinsamen Platine befindlich eingechoben werden können.

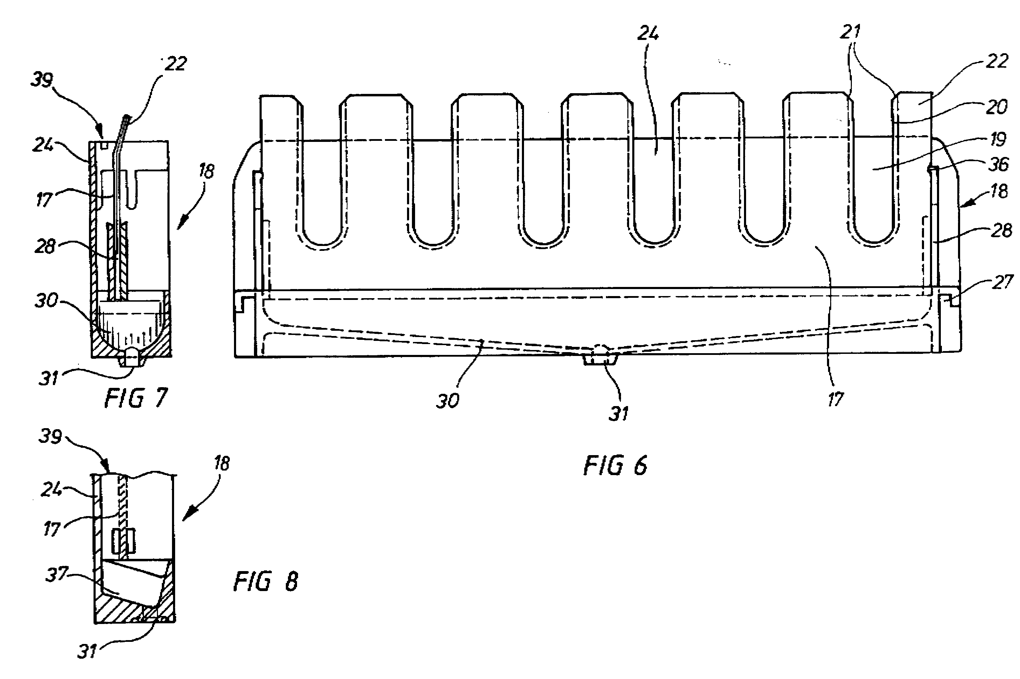

In Figur 5 ist der Aufnahmekamm 17 dargestellt, der um 180° gedreht in den Aufnahmebehälter 18 nach Figur 4 eingeführt wird.

Der Aufnahmekamm 17 weist hierbei Kanten 32, welche in Längsführungen 29 des Aufnahmebehälters 18 eingeführt werden, wobei am Aufnahmebehälter 18 Halterungen 28 ausgebildet sind.

Nach dem Einführen des Aufnahmekamms 17 in den Aufnahmebehälter 18 wird dieser Aufnahmebehälter 18 selbst in die Grundplatte 7 nach Figur 3 nach Art eines Einschubs eingeschoben, wobei die Führungen 27 des Aufnahmebehälters 18 in Führungen 26 der Grundplatte 7 eingeschoben werden.

In der Darstellung nach Figur 4 wird Aufnahmebehälter 18 hierbei um 180° gedreht, so daß die Rückwand 24 des Aufnahmebehälters 18 gegenüber der Lichtquelle 2 angeordnet ist und bei eingeschobenem Aufnahmekamm 17 nach Figur 7 und Figur 9 hierbei ein Aufnahmespalt 39 für einzuschiebende Instrumente bzw. Zahnbürsten gebildet wird.

Aus Figur 4 in Verbindung mit Figur 6 ist weiterhin ersichtlich, daß der Aufnahmebehälter 18 wannenförmig ausgebildet ist, und insbesondere nach den Figuren 7 und 8 eine Wanne 30 bzw. 37 mit einer Ablauföffnung 31 ausbildet.

In Figur 5 ist ersichtlich, daß an den Zinken 22 des Aufnahmekamms 17 Führungen 20 ausgebildet sind sowie Einlaufschrägen 21 an den ZInken 22, um das Einführen, insbesondere von Zahnbürsten, zu erleichtern.

Figur 6 zeigt bei sonst gleichen Bezugszeichen den in den Aufnahmebehälter 18 eingeschobenen Aufnahmekamm 17, wobei ersichtlich ist, daß an der Halterung 28 des Aufnahmebehälters 18 eine Rastung 36 ausgebildet ist, wo der Aufnahmekamm 17 mit entsprechenden Einkerbungen oder dergleichen verrastet.

Aus den Figuren 7 und 8 wird im Querschnitt vom Aufnahmebehälter 18 und Aufnahmekamm 17 besonders deutlich, daß in Verbindung mit der wannenförmigen Ausbildung des Aufnahmebehälters 18 ein Aufnahmespalt 39 gebildet wird, wobei die Zinken 22 des Aufnahmekamms 17 in Richtung zur Grundplatte 7 geneigt verlaufen, um das Einführen von Instrumenten oder Zahnbürsten oder dergleichen zu erleichtern. Die Zahnbürsten 4 oder dergleichen werden dann zwischen der Rückwand 24 des Aufnahmebehälters und dem Aufnahmekamm 17 in den gebildeten Aufnahmespalt 39 eingeführt, wobei der Borstenkopf 5 nach Figur 1 sich klemmend im Bereich des Aufnahmespaltes 39 abstützt, insbesondere an Führungen 20 des Aufnahmekamms 17 nach Figur 5.

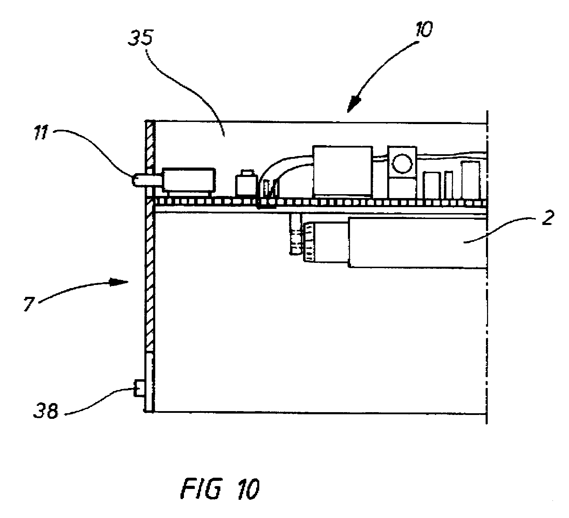

Aus der Darstellung des Querschnitts der Vorrichtung nach Figur 9 wird deutlich, daß beim Anschwenken des Gehäuses 1 an die Grundplatte 7 über Gelenkzapfen 38 nach Figur 10 insgesamt ein geschlossener Entkeimungsraum gebildet wird, wobei innerhalb dieses Entkeimungsraumes nach Figur 9 der Aufnahmebehälter 18 mit dem darin angeordneten Aufnahmekamm 17 angeordnet ist. Der Aufnahmespalt 39 für Instrumente oder Zahnbürsten oder dergleichen ist hierbei über Öffnungen 3 innerhalb des Gehäuses 1 von oben her zugänglich.

Die Lichtquelle 2 befindet sich nach Figur 9 einschließlich der Elektronik in einem Aufnahmeschacht 35 der Grundplatte 7, wobei in der Grundplatte 7 eine Lichtöffnung 34 angeordnet ist, durch welche die UV-Strahlung in das Gehäuse 1 austritt. Der Aufnahmebehälter 18 mit dem Aufnahmekamm 17 und den nicht dargestellten, darin eingesteckten ärztlichen Instrumenten oder Zahnbürsten oder dergleichen befinden sich nach Art eines Einschubes sehr dicht an der Lichtquelle 2, so daß in dieser Art eine schattenfreie, vorteilhafte UV-Bestrahlung erfolgt.

Aus der Figur 9 sind weiterhin die Sensoren 16 ersichtlich, welche insbesondere im Bereich der Öffnungen 3 das Innere des Gehäuses 1 bestreichen, wodurch mit Auslösung einer Bewegung, z. B. durch Einführen oder Herausziehen einer Zahnbürste die UV-Lichtquelle 2 über die in Figur 2 eingeschaltete Elektronik eingeschaltet wird.

Aus Figur 9 in Verbindung mit Figur 10 ist weiterhin der Schalter 11 aus Figur 2 ersichtlich, welcher beim Hochschwenken des Gehäuses betätigt wird und dadurch die Elektronik an die Versorgungsspannung legt.

Der Aufnahmekamm 17 nach Figur 9 ist durchlässig für UV-Strahlung ausgebildet, wodurch sich innerhalb des Aufnahmebehälters 18 mit Reflektion der Strahlung an der Rückwand 24 ein vorteilhafter Entkeimungsraum ausbildet, wobei das geschlossene Gehäuse 1 zusätzlich noch Strahlungen reflektiert und im übrigen durch die verschachtelte Anordnung des Aufnahmebehälters 18 innerhalb des Gehäuses 1 ein Raum geschaffen wird, wo sich durch die UV-Strahlung gebildetes Ozon vorteilhaft ansammelt.

In Figur 10 ist in dem Aufnahmeschacht 35 der Grundplatte 7 auf einer Platine die Elektronik und insbesondere die Steuerungseinrichtung 10 angeordnet, wobei die Funktion aus Figur 2 näher ersichtlich wird.

Ausgehend von dem Stecknetzgerät 9 über den Schalter 11 werden zunächst ein Zähler 13 sowie die Sensorelektronik 23 und der Spannungswandler 14 unter Spannung gesetzt.

Die Zahnbürsten 4 werden so in dem Aufnahmespalt 39 gehalten, daß ihre Borsten zur UV-Lichtquelle 2 zeigen. Diese oder andere Instrumente sind damit den ultravioletten Strahlen ausgesetzt und werden dadurch keimfrei gemacht und auch keimfrei gehalten.

Die UV-Lichtquelle 2 ist mit dem Spannungswandler 14 verbunden, wobei in dem Spannungswandler 14 mit höherer Frequenz die Versorungsspannung 2 der UV-Lichtquelle 2 erzeugt wird.

Der Zähler 13 ist ein für die Zeitüberwachung eingesetzter programmierbarer Rechner, der durch das Einschalten der Vorrichtung in Betrieb gesetzt wird und nach vorgegebener Zeit, z. B. nach einer Stunde, die UV-Lichtquelle 2 wieder ausschaltet. Aus Figur 2 ist weiterhin die Sensorelektronik 23 ersichtlich, wo über die Sensoren 16, bestehend aus Infrarotsendediode und Infrarotempfangsdiode, die Zahnbürsten 4 angeordnet sind. Bei einer Bewegung der Zahnbürsten 4, sei es durch Hineinschieben in die Vorrichtung oder durch das Herausziehen aus derselben, spricht die Sensorelektornik 23 an und schaltet den Zähler 13 ein, welcher wiederum den Spannungswandler 14 startet und hierdurch die Lichtquelle 2 in Betrieb genommen wird.

Es wird mit einer UV-Lichtquelle gearbeitet, die Licht mit einer Wellenlänge von 253,7 nm erzeugt. Vorteilhaft ist aber auch eine zweistufig arbeitende UV-Lichtquelle einsetzbar, durch welche im Wellenlängenbereich von 180 bis 200 nm Ozon gebildet wird. Durch das Bestrahlen und Ozonieren der Borstenköpfe wird eine breitgefächterte Entkeimung erreicht, insbesondere in Verbindung mit der wannenförmigen Ausbildung des Aufnahmebehälters 18 und der Mehrfachreflektion der erzeugten UV-Strahlung im Bereich der Verschachtelung zwischen Aufnahmebehälter 18 und Gehäuse 1 bzw. zwischen der Rückwand 24 der Grundplatte 7 und dem Aufnahmebehälter 18.

Die Vorrichtung zeichnet sich durch einen minimalen Stromverbrauch aus und arbeitet im für Badezimmer gefahrlosen Niederspannungsbereich.

Die beschriebene Vorrichtung zum Keimfreimachen und Keimfreihalten von Zahnbürsten ist nicht nur für private Haushalte gedacht, sie kann vielmehr auch in zahnärztlichen Praxen und in Hotelzimmern verwendet werden.

Es liegt im Rahmen der Erfindung, die beschriebene Vorrichtung oder eine ähnlich aufgebaute Vorrichtung zum Keimfreimachen und Keimfreihalten von anderen Instrumenten, welche der Behandlung oder der Pflege des menschlichen oder tierischen Körpers dienen, zu verwenden. In Frage kommen dabei neben ärztlichen bzw. zahnärztlichen Instrumenten auch Coiffeur-Instrumente, wie Rasiermesser und dergleichen sowie Hand- und Fußpflege-Instrumente.

### ZEICHNUNGS-LEGENDE

- 1: Gehäuse
- 2: Lichtquelle
- 3: Öffnung
- 4: Zahnbürste
- 5: Borstenkopf
- 6: Griff
- 7: Grundplatte
- 8: Kabel
- 9: Stecknetzgerät
- 10: Steuerungseinrichtung
- 11: Schalter
- 13: Zähler
- 14: Spannungsumwandler
- 16: Sensor
- 17: Aufnahmekamm
- 18: Aufnahmebehälter
- 19: Aufnahmeöffnung
- 20: Führung
- 21: Einlaufschräge
- 22: Zinken
- 23: Sensorelektronik
- 24: Rückwand
- 25: Schenkel
- 26: Führung
- 27: Führung
- 28: Halterung
- 29: Längsführung
- 30: Wanne
- 31: Ablauföffnung
- 32: Kanten
- 33: Wand
- 34: Lichtöffnung
- 35: Aufnahmeschacht
- 36: Rastung
- 37: Wanne
- 38: Gelenkzapfen
- 39: Aufnahmespalt

## Patentansprüche

1. Vorrichtung zum Entkeimen von Toiletteninstrumenten oder ärztlichen Instrumenten oder dergleichen, insbesondere Zahnbürsten, durch ultraviolette Strahlung, wobei die Instrumente bzw. Zahnbürsten in einem Gehäuse in Verbindung mit einer Grundplatte angeordnet sind und von einer Lichtquelle, die UV-Strahlung erzeugt, angestrahlt werden, **dadurch gekennzeichnet**, daß an die Grundplatte (7) ein Aufnahmebehälter (18) mit einem Aufnahmekamm (17) für die Instrumente bzw. Zahnbürsten (4) ansetzbar ist, und daß das Gehäuse (1) mit Öffnungen (3) schwenkbar und einrastbar an Schenkeln (25) der Grundplatte (7) angeordnet ist.

2. Vorrichtung nach Anspruch 1, **dadurch gekennzeichnet**, daß die Grundplatte (7) eine Wand (33) mit einer Öffnung (34) für die UV-Lichtquelle (2) aufweist, wobei der Aufnahmebehälter (18) mit dem Aufnahmekamm (17) nach Art eines Einschubes an die Lichtquelle (2) herangeführt wird.

3. Vorrichtung nach Anspruch 1, **dadurch gekennzeichnet**, daß die Schenkel (25) der Grundplatte (7) Führungen (26) aufweisen, in welche der Aufnahmebehälter (18) über weitere Führungen (27) einschiebbar ist.

4. Vorrichtung nach Anspruch 1, **dadurch gekennzeichnet**, daß der Aufnahmebehälter (18) seitliche Halterungen (28) mit Längsführungen (29) und eine Rastung (36) aufweist, in welche der Aufnahmekamm (17) einschiebbar ist.

5. Vorrichtung nach Anspruch 1, **dadurch gekennzeichnet**, daß der Aufnahmebehälter (18) eine hochgezogene Wand (24) aufweist, wo in Verbindung mit dem eingeschobenen Aufnahmekamm (17) ein Aufnahmespalt (39) für Instrumente bzw. die Zahnbürsten (4) gebildet wird.

6. Vorrichtung nach Anspruch 1, **dadurch gekennzeichnet**, daß der Aufnahmekamm (17) an den Zinken (22) Führungen (20) für Instrumente bzw. den Borstenkopf (5) einer Zahnbürste (4) aufweist.

7. Vorrichtung nach Anspruch 1, **dadurch gekennzeichnet**, daß der Boden des Aufnahmebehälters (18) wannenförmig mit einer Ablauföffnung (31) ausgebildet ist.

8. Vorrichtung nach Anspruch 1, **dadurch gekennzeichnet**, daß an der Grundplatte (7) in seitlicher Anordnung mit Ausleuchtungen des Innenraumes des Gehäuses (1) im Bereich des Aufnahmebehälters (18) und der Öffnungen (3) Infrarotsensoren (16), bestehend aus Sender- und Empfängerdioden, angeordnet sind.

9. Vorrichtung nach Anspruch 1, **dadurch gekennzeichnet**, daß die Grundplatte (7) einen Aufnahmeschacht (35) ausbildet, in welchen die Elektronik mit der Steuerungseinrichtung (10) und die UV-Lichtquelle (2) einschiebbar sind.

10. Vorrichtung nach Anspruch 1, **dadurch gekennzeichnet**, daß seitlich an der Grundplatte (7) in einem Durchbruch ein Schalter (11) angeordnet ist, welcher durch das Schwenken des Gehäuses (1) betätigt wird.

11. Vorrichtung nach Anspruch 9, **dadurch gekennzeichnet**, daß die Steuerungseinrichtung (10) mit einem Zähler (13) ausgestattet ist, der die eingeschaltete Lichtquelle (2) nach einer vorgegebenen Zeit ausschaltet.

12. Vorrichtung nach Anspruch 9, **dadurch gekennzeichnet**, daß die Steuerungseinrichtung (10) mit einem Thermostat ausgestattet ist, der die eingeschalteten Lichtquellen (2) beim Überschreiten einer vorgegebenen Temperatur ausschaltet und bei deren Unterschreiten wieder einschaltet.

13. Vorrichtung nach Anspruch 11, **dadurch gekennzeichnet**, daß der Zähler (13) durch manuelles Einschalten der Vorrichtung in Betrieb gesetzt wird.

14. Vorrichtung nach Anspruch 8, **dadurch gekennzeichnet**, daß die Sensorabtastung auf induktivem oder kapazitivem Weg erfolgt.

15. Vorrichtung nach Anspruch 8, **dadurch gekennzeichnet**, daß der bzw. die Sensoren (16) auf Feuchtigkeit ansprechen.

16. Vorrichtung nach Anspruch 8, **dadurch gekennzeichnet**, daß der bzw. die Sensoren (16) das Einstecken oder Einlegen eines Instrumentes (4) mittels Ultraschall wahrnehmen.

17. Vorrichtung nach den Ansprüchen 1 und 2, **dadurch gekennzeichnet**, daß die Lichtquelle (2) zweistufig arbeitet und UV-Licht im Wellenlängenbereich von 180 bis 200 nm und von 253,7 nm erzeugt, wobei im Wellenlängenbereich von 180 bis 200 nm Ozon gebildet wird.

18. Vorrichtung nach Anspruch 9, **gekennzeichnet durch** ein Stecknetzgerät, welches die Betriebsspannung von 220 oder 110 Volt auf 8 bis 12 Volt, vorzugsweise auf 8 Volt transformiert.

19. Vorrichtung nach Anspruch 1, **dadurch gekennzeichnet**, daß der Aufnahmekamm (17) aus strahlungsdurchlässigem Material ausgebildet ist.

## Claims

1. A device for sterilizing toilet instruments or medical instruments or the like, in particular toothbrushes, by ultraviolet radiation, in which the instruments or toothbrushes are arranged in a housing in connection with a base plate and are irradiated from a light source which produces UV radiation, characterised in that a holding container (18) with a holding comb (17) for the instruments or toothbrushes (4) is able to be joined on to the base plate (7), and that the housing (1) with openings (3) is arranged so as to be pivotable and able to be engaged on shanks (25) of the base plate (7).

2. A device according to Claim 1, characterised in that the base plate (7) has a wall (33) with an opening (34) for the UV light source (2), in which the holding container (18) with the holding comb (17) is brought up to the light source (2) in the manner of an insert.

3. A device according to Claim 1, characterised in that the shanks (25) of the base plate (7) have guides (26), into which the holding container (18) is able to be introduced via further guides (27).

4. A device according to Claim 1, characterised in that the holding container (18) has lateral mountings (28) with longitudinal guides (29) and a catch (36), into which the holding comb (17) is able to be introduced.

5. A device according to Claim 1, characterised in that the holding container (18) has a raised wall (24), where in connection with the inserted holding comb (17) a holding slot (39) for instruments or the toothbrushes (4) is formed.

6. A device according to Claim 1, characterised in that the holding comb (17) has on the prongs (22) guides (20) for instruments or for the brush head (5) of a toothbrush (4).

7. A device according to Claim 1, characterised in that the floor of the holding container (18) is constructed in a trough shape with an outlet opening (31).

8. A device according to Claim 1, characterised in that on the base plate (7) in lateral arrangement with illuminations of the interior of the housing (1) in the region of the holding container (18) and of the openings (3) infrared sensors (16), consisting of transmitter- and receiver diodes, are arranged.

9. A device according to Claim 1, characterised in that the base plate (7) forms a holding shaft (35), into which the electronics are able to be introduced, with the control apparatus (10) and the UV light source (2).

10. A device according to Claim 1, characterised in that laterally on the base plate (7) a switch (11) is arranged in an aperture, which switch is actuated by the pivoting of the housing (1).

11. A device according to Claim 9, characterised in that the control apparatus (10) is equipped with a counter (13), which switches off the switched-on light source (2) after a predetermined time.

12. A device according to Claim 9, characterised in that the control apparatus (10) is equipped with a thermostat, which switches off the switched-on light sources (2) on exceeding a predetermined temperature, and switches it on again on falling below this temperature.

13. A device according to Claim 11, characterised in that the counter (13) is set in operation by switching the device on manually.

14. A device according to Claim 8, characterised in that the sensor scanning takes place inductively or capacitively.

15. A device according to Claim 8, characterised in that the sensor or sensors (16) respond to moisture.

16. A device according to Claim 8, characterised in that the sensor or sensors (16) detect the insertion or placement of an instrument (4) by means of ultrasonics.

17. A device according to Claims 1 and 2, characterised in that the light source (2) operates in two stages and produces UV light in the wavelength range from 180 to 200 nm and of 253.7 nm, in which in the wave length range of 180 to 200 nm ozone is formed.

18. A device according to Claim 9, characterised by a plug power unit, which transforms the operating voltage from 220 or 110 volts to 8 to 12 volts, preferably to 8 volts.

19. A device according to Claim 1, characterised in that the holding comb (17) is constructed from material which is permeable to radiation.

## Revendications

1. Dispositif pour aseptiser des instruments de toilette, des instruments médicaux ou des instruments similaires, notamment des brosses à dents, grâce à un rayonnement ultraviolet, les instruments ou les brosses à dents étant disposés dans un boîtier associé à une plaque de montage et étant éclairés par une source lumineuse produisant un rayonnement UV, caractérisé en ce qu'un récipient collecteur (18) pourvu d'un peigne collecteur (17) pour les instruments ou les brosses à dents (4) peut être fixé à la plaque de montage (7), et en ce que le boîtier (1) pourvu d'ouvertures (3) est disposé pour pouvoir pivoter et s'encliqueter au niveau d'ailes (25) de la plaque de montage (7).

2. Dispositif selon la revendication 1, caractérisé en ce que la plaque de montage (7) comporte une paroi (33) pourvue d'une ouverture (34) pour la source lumineuse UV (2), le récipient collecteur (18) avec le peigne collecteur (17) étant rapproché de la source lumineuse (2) à la manière d'un tiroir.

3. Dispositif selon la revendication 1, caractérisé en ce que les ailes (25) de la plaque de montage (7) Comportent des guidages (26) dans lesquels le récipient collecteur (18) peut être inséré par l'intermédiaire d'autres guidages (27).

4. Dispositif selon la revendication 1, caractérisé en ce que le récipient collecteur (18) comporte des fixations latérales (28) pourvues de guidages longitudinaux (29), et un crantage (36) dans lequel le peigne collecteur (17) peut être insére.

5. Dispositif selon la revendication 1, caractérisé en ce que le récipient collecteur (18) comporte une paroi (24) dressée vers le haut qui définit, en association avec le peigne collecteur (17) inséré, une fente de logement (39) pour des instruments ou pour les brosses à dents (4).

6. Dispositif selon la revendication 1, caractérisé en ce que le peigne collecteur (17) comporte, au niveau des dents (22), des guidages (20) pour des instruments ou pour la tête à poils (5) d'une brosse à dents (4).

7. Dispositif selon la revendication 1, caractérisé en ce que le fond du récipient collecteur (18) est en forme de cuvette et comporte une ouverture d'écoulement (31).

8. Dispositif selon la revendication 1, caractérisé en ce qu'il est prévu, au niveau de la plaque de montage (7), des détecteurs à infrarouge (16) formés de diodes émettrices et réceptrices, qui sont disposés latéralement, avec des éclairages de l'intérieur du boîtier (1), dans la zone du récipient collecteur (18) et des ouvertures (3).

9. Dispositif selon la revendication 1, caractérisé en ce que la plaque de montage (7) forme une gaine collectrice (35) dans laquelle le système électronique comportant le dispositif de commande (10), et la source lumineuse UV (2) peuvent être insérés.

10. Dispositif selon la revendication 1, caractérisé en ce qu'il est prévu, disposé dans un ajour, sur le côté de la plaque de montage (7), un commutateur (11) qui est actionné grâce au pivotement du boîtier (1).

11. Dispositif selon la revendication 9, caractérisé en ce que le dispositif de commande (10) est équipé d'un compteur (13) qui, après un certain temps, éteint la source lumineuse (2) allumée.

12. Dispositif selon la revendication 9, caractérisé en ce que le dispositif de commande (10) est équipé d'un thermostat qui éteint les sources lumineuses (2) allumées, lorsqu'une température prédéfinie est dépassée, et qui les rallume au-dessous de cette température.

13. Dispositif selon la revendication 11, caractérisé en ce que le compteur (13) est mis en marche grâce à la mise en circuit manuelle du dispositif.

14. Dispositif selon la revendication 8, caractérisé en ce que l'exploration des détecteurs se fait par voie inductive ou capacitive.

15. Dispositif selon la revendication 8, caractérisé en ce que le ou les détecteurs (16) réagissent à l'humidité.

16. Dispositif selon la revendication 8, caractérisé en ce que le ou les détecteurs (16) constatent à l'aide d'ultrasons l'introduction ou la mise en place d'un instrument (4).

17. Dispositif selon les revendications 1 et 2, caractérisé en ce que la source lumineuse (2) fonctionne sur deux degrés et génère de la lumière UV dans la plage de longueur d'ondes allant de 180 à 200 nm, et de 253,7 nm, de l'ozone étant formée dans la plage de longueurs d'ondes de 180 à 200 nm.

18. Dispositif selon la revendication 9, caractérisé par un appareil d'alimentation enfichable qui transforme la tension de régime de 220 ou 110 volts en 8 à 12 volts, de préférence en 8 volts.

19. Dispositif selon la revendication 1, caractérisé en ce que le peigne collecteur (17) est réalisé en un matériau perméable au rayonnement.
